# EUROPÄISCHE PATENTSCHRIFT

(11) **EP 1 017 688 B1**
(45) Veröffentlichungstag und Bekanntmachung des Hinweises auf die Patenterteilung: **10.04.2002**
(21) Anmeldenummer: 98948912.5
(22) Anmeldetag: 05.09.1998
(51) Int. Cl.: C07D 307/88, C25B 3/04

(54) **VERFAHREN ZUR HERSTELLUNG VON PHTHALIDEN**
METHOD FOR PRODUCING PHTHALIDES
PROCEDE DE PRODUCTION DE PHTALIDES

(30) Priorität: 19.09.1997 DE 19741423
(43) Veröffentlichungstag der Anmeldung: 12.07.2000
(73) Patentinhaber: BASF AKTIENGESELLSCHAFT, 67056 Ludwigshafen (DE)
(72) Erfinder: PÜTTER, Hermann, D-67433 Neustadt (DE); BAUMANN, Dieter, D-69190 Walldorf (DE); HANNEBAUM, Heinz, D-67069 Ludwigshafen (DE)
(86) Internationale Anmeldenummer: EP9805626
(87) Internationale Veröffentlichungsnummer: WO9915514

(56) Entgegenhaltungen:
- EP-B- 0 902 846
- WO-A-97/43464
- DE-A- 2 144 419
- DE-A- 2 510 920

## Beschreibung

Die vorliegende Erfindung betrifft ein neues Verfahren zur Herstellung von Phthaliden besonders hoher Reinheit, bei dem man
I. in einem Elektrolyten gelöste Phthalsäure oder Phthalsäurederivate, bei denen die Carboxygruppen durch Einheiten ersetzt sein können, die von Carboxygruppen in einer Kondensationsreaktion ableitbar sind und eines oder mehrere der Wasserstoffatome der o-Phenylen-Einheit durch inerte Reste substituiert sein können, an einer Kathode in einer ungeteilten Elektrolysezelle reduziert,
II. wenn der Umsatz so weit fortgeschritten ist, daß das Molverhältnis (M), gebildet aus dem Anteil an Phthalid und der Summe aus dem Anteil an Phthalid und der Phthalsäure oder den Phthalsäurederivaten im Elektrolyten 0,8 :1 bis 0,995:1 beträgt, den Elektrolyten aus der Elektrolysezelle austrägt und
III. die Phthalide, nach destillativer Aufar-5 beitung des Elektrolyten, aus dem Elektrolyt kristallisiert und von der Mutterlauge abtrennt.

Phthalide werden insbesondere als Zwischenprodukte für die Herstellung von Pflanzenschutzmitteln benötigt.

Ein elektrochemisches Verfahren zur Herstellung von Phthaliden ist aus der DE A-2 144 419 bekannt. Hierbei wird Ammoniumphthalamat in wässeriger Lösung mit einem Anteil organischer Lösungsmittel bis 50% bei Temperaturen bis 65°C an Metallen mit einer Wasserstoffüberspannung größer als Cu, z.B. Blei, kathodisch reduziert. Unter diesen Bedingungen gelingt die Herstellung von Phthaliden in befriedigenden Ausbeuten, wenn man die Reduktion in geteilten Elektrolysezellen vornimmt.

Die Herstellung von besonders reinen Phthaliden ist in der DE-A-2 510 920 beschrieben. Nach dieser Lehre reduziert man ammoniakalische wässerige Lösungen von Phthalsäure oder Phthalsäureanhydrid kathodisch bei Temperaturen von bis zu 100°C an Metallen mit einer Wasserstoffüberspannung größer als Cu. Das Verfahren erfordert ebenfalls die Anwendung geteilter Elektrolysezellen. Zur Abtrennung des Phthalids aus dem Elektrolysegemisch wird es gegebenenfalls nach Abtrennung von überschüssigem Ammoniak bei einer Temperatur von 35 bis 100°C angesäuert und das ausgefallene Phthalid abgetrennt.

Nachteilig an den beschriebenen Verfahren ist jedoch der mit der Verwendung von geteilten Elektrolysezellen verbundene apparative Aufwand, da in diesem Fall 2 Zellkreise erforderlich sind. Zudem ist das Arbeiten mit 2 Zellkreisen mit folgenden weiteren Nachteilen verbunden:

Die Zellkreise müssen durch eine Membran oder ein Diaphragma getrennt werden; dies bedeutet einen Verlust an Energie durch ohmsche Wärme. Um diesen Verlust zu minimieren, wird meist wenigstens eine Kammer mit einer wäßrigen (> 80 % H₂O) Leitsalzlösung beschickt. Bei kathodischen Reduktionen ist dies der Anolyt. Der Zwang zu diesem Vorgehen engt die Freiräume zur Nutzung der Anodenreaktion stark ein. Normalerweise wird als Anodenprodukt lediglich Sauerstoff erzeugt.

Die Herstellung von Phthaliden mittels elektrochemischer Reduktion von Phthalsäurederivaten in einer ungeteilten Elektrolysezelle wird in der noch nicht vorveröffentlichen DE Patentschrift mit dem Aktenzeichen 19618854 vorgeschlagen. In dieser Schrift wird weiterhin vorgeschlagen, das Phthalid durch Umkristallisation zu reinigen. Konkrete Angaben, bis zu welchem Umsatz die Elektrolyse der Phthalsäurederivate durchgeführt wird, bevor sie beendet wird, finden sich in dieser Schrift nicht. Auch in den Beispielen ist der Umsatz für den Fachmann nicht implizit offenbart.

Die nach den vorbeschriebenen Methoden hergestellten Phthalide weisen zwar eine recht hohe Reinheit auf, die auch für die meisten Anwendungszwecke ausreicht. In einigen Fällen werden jedoch die Phthalide in einer Reinheit benötigt, die man mit den vorbekannten Methoden gar nicht oder nur mit großem Aufwand erreichen kann.

Insbesondere bei der Verwendung von Phthalsäuredimethylestern und deren kernsubstituierten Derivaten besteht das Problem darin, daß sich das Phthalid und sein Ausgangsprodukt destillativ kaum voneinander trennen lassen. Prinzipiell kann man dieses Reinigungsproblem zwar dadurch umgehen, daß man die Elektrolyse so lange durchführt, bis praktisch die gesamte Menge an Ausgangsprodukt umgesetzt ist. Beispielsweise sollte zur Erzeugung einer 98 %igen Qualität das Ausgangsprodukt soweit umgesetzt sein, daß das Gewichtsverhälnis Produkt zu Edukt mindestens 98:2 und das Molverhältnis 0,98 zu 1 beträgt. Bei Verwendung von Phthalsäuredimethylester (MG 194) muß demnach ein Gewichtsverhältnis Produkt zu Edukt von 99:1 angestrebt werden. Diese Lösung des Reinigungsproblems ist jedoch mit dem Nachteil verbunden, daß die Selektivität der Reaktion stark zurückgeht und in erheblichem Umfang unbrauchbare Nebenprodukte gebildet werden.

Die Aufgabe bestand deshalb darin, eine Methode bereitzustellen, mit der man Phthalide in hoher Reinheit, guten Ausbeuten und nach einem wirtschaftlichen und wenig aufwendigen technischen Verfahren bereitstellen kann.

Demgemäß wurde das eingangs beschriebene Verfahren gefunden.

Als Ausgangsverbindugen für die Herstellung der Phthalide werden insbesondere solche der allgemeinen Formel (I) 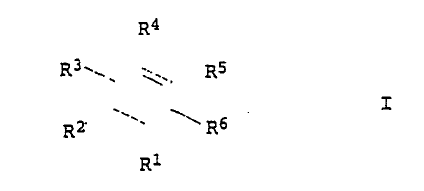 einsetzt, in der die Substituenten die folgende Bedeutung haben:
- R¹, R², R³ und R⁴:: unabhängig voneinander Wasserstoff, C₁- bis C₄-Alkyl oder Halogen
- R⁵, R⁶:: a) unabhängig voneinander -COOH oder COOX, wobei X für C₁- bis C₄-Alkyl steht,
b) einer der Substituenten R⁵ oder R⁶-COONY₄ und der andere Substituent CONH₂, wobei Y für C₁- bis C₄-Alkyl oder Wasserstoff steht,
c) R⁵ und R⁶ zusammen -CO-O-CO-.

Besonders bevorzugt sind die Derivate der Phthalsäure, bei denen R¹, R², R³ und R⁴ Wasserstoff bedeutet und darunter insbesondere die Phthalsäuredi(C₁- bis C₃-alkyl)-ester, vor allem der Phthalsäuredimethylester.

Die elektrochemische Umsetzung dieser Ausgangsprodukte kann beispielsweise nach der Methode erfolgen, wie sie in der der DE-A-19618854 beschrieben ist.

Bei diesem Verfahren werden als Elektrodenmaterialien (sowohl Kathode als auch Anode) vor allem handelsübliche Elektroden aus Graphit oder Kohle eingesetzt.

Bei dem Elektrolyten handelt es sich üblicherweise um eine 2 bis 40 gew.-%ige Lösung der Phthalsäure oder eines Phthalsäurederivates in einem organischen Lösungsmittel oder einer Mischung aus einem organischen Lösungsmittel und Wasser, wobei die Mischung im allgemeinen weniger als 50 Gew.-%, bevorzugt weniger als 25, besonders bevorzugt weniger als 5 Gew.-% Wasser enthält.

Als organische Lösungsmittel eignen sich insbesondere aliphatische C₁- bis C₄-Alkohole, insbesondere Methanol oder Ethanol oder Mischungen derartiger Alkohole mit einem Carbonsäureamid wie Dimethylformamid oder t-Butylformamid.

Als Leitsalze enthalten die Elektrolyte beispielsweise quarternäre Ammoniumsalze, wie Tetra (C₁- bis C₄-alkyl)ammoniumhalogenide oder -tetrafluoroborate und bevorzugt Methyltributylammonium- oder Methyltriethylammonium-methylsulfat üblicherweise in Mengen von 0,4 bis 10 Gew.-%, bezogen auf den Elektrolyt.

Für den anodischen Koppelprozeß empfiehlt es sich, als anodischen Depolarisator übliche organische Verbindung einzusetzen, deren Eignung für die elektrochemische Oxidation dem Fachmann allgemein bekannt ist. Einige der anodischen Koppelprozessen werden bevorzugt in Anwesenheit eines Mediators durchgeführt. Geeignete anodische Koppelprozesse werden beispielsweise in D. Kyriakou, Modern Electroorganic Chemistry, Springer, Berlin 1994 in Kapitel 2 beschrieben.

Als anodische Koppelprozesse eignen sich insbesondere die Oxidationen von C-O- oder C-N-Einfach- oder Doppelbindungen, z.B. die Oxidation von Carbonsäuren, oder die oxidative C-C-Verknüpfung 5 insbesondere von Naphthalinen oder aktivierten CH-Gruppen sowie die Oxidation von an einen aromatischen Kern gebundenen Methylgruppen zu Aldehyden.

Als besonders günstig hat sich der Einsatz von Methylbenzol oder 0 kernsubstituierten Derivaten des Methylbenzols erwiesen, bei dem 1 bis 3 Wasserstoffatome des Phenylrestes durch C₁- bis C₆-Alkylreste oder C₁- bis C₄-Alkoxyreste ersetzt sein können. Beispiele für derartige anodische Depolarisatoren sind p-Xylol und p-tert-Butyltoluol.

Bei der Herstellung von Aldehyden als Koppelprodukten empfiehlt sich der Einsatz von den genannten Alkoholen als Lösungsmittel, da die Aldehyde acetalisiert und vor einer Weiteroxidation geschützt werden.

Was die sonstigen Verfahrensparameter wie Temperatur und Stromdichte betrifft, so sind diese unkritisch, solange sie sich im für die elektrochemische Umsetzung organischer Verbindungen üblichen Rahmen bewegen. Sie sind beispielsweise in der DE-A-2510920 näher spezifiziert.

Wenn der Umsatz so weit fortgeschritten ist, daß das Molverhältnis (M), gebildet aus dem Anteil an Phthalid und der Summe aus dem Anteil an Phthalid und der Phthalsäure oder den Phthalsäurederivaten im Elektrolyten 0,8:1 bis 0,995:1, bevorzugt 0,83:1 bis 0,99:1 und besonders bevorzugt 0,86:1 bis 0,95:1 beträgt, trägt man den Elektrolyten aus der Elektrolysezelle aus.

Die Reaktion kann sowohl diskontinuierlich als auch kontinuierlich durchgeführt werden.

Bei der kontinuierlichen Durchführung des Verfahrens wird man zweckmäßigerweise den kontinuierlichen Austrag des Elektrolyten und die kontinuierliche Ergänzung der inerten Bestandteile des Elektrolyten wie der Lösungsmittel und Leitsalze sowie der Ausgangsprodukte für die elektrochemischen Reaktion so aufeinander und auf die Reaktionsgeschwindigkeit abstimmen, daß die Konzentration aller Bestandteile des Elektrolyten weitgehend konstant bleibt. Dies gilt insbesondere für das Molverhältnis (M), das sich innerhalb des definitionsgemäßen Bereiches bewegt.

Der ausgetragene Elektrolyt (im folgenden Text "Rohphthalid" genannt) wird vor der Kristallisation destillativ aufgearbeitet. Dies geschieht bevorzugt auf folgende Weise:

Zunächst wird aus dem Elektrolyten das Lösungsmittel und anschließend eine Fraktion, die die Phthalide enthält, abdestilliert. Mit Vorteil wird die Destillation so durchgeführt, daß man neben dem Lösungsmittel und der Fraktion, die hauptsächlich die Phthalide enthält, eine weitere Fraktion separat auffängt, die hauptsächlich das Koppelprodukt enthält. Der verbleibende Destillationsrückstand enthält im allgemeinen hauptsächlich das Leitsalz.

Die Destillation des Rohphthalids erfolgt im allgemeinen bei einem Druck von 1 bis 100 mbar und einer Temperatur von 100 bis 220°C. Hierzu bedient man sich z.B. eines Dünnschichtverdampfers.

Der Destillationsrückstand, der meistens im wesentlichen aus dem Leitsalz besteht, kann in die Elektrolysezelle zurückgeführt werden.

Das gegebenenfalls auf diese Weise vorgereinigte Rohphthalid wird anschließend durch Kristallisation gereinigt.

Das verwendete Kristallisationsverfahren unterliegt keiner Beschränkung. Die Kristallisation kann kontinuierlich oder diskontinuierlich, einstufig oder mehrstufig durchgeführt werden.

Hierbei wird bevorzugt ohne Zusatz eines Hilfsstoffs, insbesondere ohne Zusatz eines organischen Lösungsmittels, gearbeitet.

Vorzugsweise erfolgt die Kristallisation einstufig. In einer anderen bevorzugten Ausführungsform der Erfindung wird die Kristallisation als fraktionierte Kristallisation durchgeführt.

Üblicherweise werden bei fraktionierter Kristallisation alle Stufen, die ein Kristallisat erzeugen, das reiner ist als das zugeführte Rohphthalid, Reinigungsstufen genannt und alle anderen Stufen Abtriebsstufen genannt. Zweckmäßigerweise werden mehrstufige Verfahren hierbei nach dem Gegenstromprinzip betrieben, bei dem nach der Kristallisation in jeder Stufe das Kristallisat von der Mutterlauge abgetrennt wird und dieses Kristallisat der jeweiligen Stufe mit dem nächsthöheren Reinheitsgrad zugeführt wird, während der Kristallisationsrückstand der jeweiligen Stufe mit dem nächstniedrigen Reinheitsgrad zugeführt wird.

Vorteilhafterweise liegt die Temperatur der Lösung oder Schmelze während der Kristallisation zwischen -10 und 75°C, insbesondere zwischen 20 und 70°C. Der Feststoffgehalt im Kristallisator liegt üblicherweise zwischen 0 und 70g bevorzugt zwischen 30 und 60g pro 100g Einsatz.

In einer weiteren vorteilhaften Ausführung der Erfindung erfolgt die Kristallisation in Apparaten, in denen die Kristalle im Kristallisationsapparat an gekühlten Flächen aufwachsen, d.h. im Apparat fixiert sind (z.B. Schichtkristallisationsverfahren der Fa. Sulzer Chemtech (Schweiz) oder Statisches Kristallisationsverfahren der Fa. BEFS PROKEM (Frankreich).

Weiterhin kann die Kristallisation durch Kühlung von Apparate-wänden oder durch Verdampfung einer Lösung des Rohphthalids im Vakuum erfolgen. Geeignet sind hierfür besonders 5 bis 30 gew%ige Lösungen des Rohphthalids in Methanol, wobei es sich bei dem Methanol auch um das als Lösungsmittel im Elektrolyten eingesetzte Methanol handeln kann.

Bei der Kristallisation durch Kühlung wird die Wärme über Kratzkühler, die mit einem Rührkessel oder einem Behälter ohne Rührwerk verbunden sind, abgeführt. Der Umlauf der Kristallsuspension wird hierbei durch eine Pumpe gewährleistet. Daneben besteht auch die Möglichkeit, die Wärme über die Wand eines Rührkessels mit wandgängigem Rührer abzuführen. Eine weitere bevorzugte Ausführungsform bei der Kühlungskristallisation ist die Verwendung von Kühlscheibenkristallisatoren, wie sie z.B. von der Fa. Gouda (Holland) hergestellt werden. Bei einer weiteren geeigneten Variante zur Kristallisation durch Kühlung wird die Wärme über herkömmliche Wärmeüberträger (bevorzugt Rohrbündel- oder Plattenwärmeüberträger) abgeführt. Diese Apparate besitzen im Gegensatz zu Kratzkühlern, Rührkesseln mit wandgängigen Rührern oder Kühlkristallscheiben keine Vorrichtung zur Vermeidung von Kristallschichten auf den wärmeübertragenden Flächen. Wird im Betrieb ein Zustand erreicht, bei dem der Wärmedurchgangswiderstand durch Kristallschichtbildung einen zu hohen Wert annimmt, erfolgt die Umschaltung auf einen zweiten Apparat. Während der Betriebszeit des zweiten Apparats wird der erste Apparat regeneriert (vorzugsweise durch Abschmelzen der Kristallschicht oder Durchspülen des Apparats mit ungesättigter Lösung). Wird im zweiten Apparat ein zu hoher Wärmedurchgangswiderstand erreicht, schaltet man wieder auf den ersten Apparat um usw. Diese Variante kann auch mit mehr als zwei Apparaten im Wechsel betrieben werden. Außerdem kann die Kristallisation durch eine herkömmliche Verdampfung der Lösung im Vakuum erfolgen.

Zur Abtrennung der Mutterlauge von dem auskristallisierten Phthalid eignen sich alle bekannten Verfahren der Fest-Flüssig-Trennung. In einer bevorzugten Ausführungsform der Erfindung werden die Kristalle durch Filtrieren und/oder Zentrifugieren von der Mutterlauge abgetrennt. Vorteilhafterweise wird dem Filtrieren oder Zentrifugieren eine Voreindickung der Suspension, zum Beispiel durch Hydrozyklon(e), vorgeschaltet. Zum Zentrifugieren eignen sich alle bekannten Zentrifugen, die diskontinuierlich oder kontinuierlich arbeiten. Am vorteilhaftesten werden Schubzentrifugen verwendet, die ein- oder mehrstufig betrieben werden können. Daneben eignen sich auch Schneckensiebzentrifugen oder Schneckenaustragszentrifugen (Dekanter). Eine Filtration erfolgt vorteilhafterweise mittels Filternutschen, die diskontinuierlich oder kontinuierlich, mit oder ohne Rührwerk, oder mittels Bandfilter betrieben werden. Allgemein kann das Filtrieren unter Druck oder im Vakuum erfolgen.

Während und/oder nach der Fest-Flüssig-Trennung können weitere Verfahrensschritte zur Steigerung der Reinheit der Kristalle bzw. des Kristallkuchens vorgesehen werden. In einer besonders vorteilhaften Ausgestaltung der Erfindung schließt sich nach dem Abtrennen der Kristalle von der Mutterlauge ein ein- oder mehrstufiges Waschen und/oder Schwitzen der Kristalle oder des Kristallkuchens an.

Beim Waschen liegt die Waschflüssigkeitsmenge geeigneterweise zwischen 0 und 500 g Waschflüssigkeit/100 g Kristallisat, vorzugsweise zwischen 30 und 200 g Waschflüssigkeit/100 g Kristallisat.

Geeignete Waschflüssigkeiten sind beispielsweise
a) das Lösungsmittel, wenn aus einem Lösungsmittel kristallisiert wird,
b) flüssiges Reinprodukt oder
c) flüssiges Einsatzprodukt.

Das Waschen kann in hierfür üblichen Apparaten erfolgen. Vorteilhafterweise werden Waschkolonnen, in denen die Abtrennung der Mutterlauge und das Waschen in einem Apparat erfolgen, Zentrifugen, die ein- oder mehrstufig betrieben werden können, oder Filternutschen oder Bandfilter verwendet. Das Waschen kann auf Zentrifugen oder Bandfiltern ein- oder mehrstufig durchgeführt werden. Hierbei kann die Waschflüssigkeit im Gegenstrom zum Kristallkuchen geführt werden.

Beim Schwitzen handelt es sich um ein lokales Abschmelzen verunreinigter Bereiche. Vorteilhafterweise beträgt die Schwitzmenge 0,1 bis 90 g abgeschmolzenes Kristallisat/100 g Kristallisat vor dem Schwitzen, vorzugsweise 5 bis 35 g abgeschmolzenes Kristallisat/100 g Kristallisat. Besonders bevorzugt ist die Durchführung des Schwitzens auf Zentrifugen oder Bandfiltern. Auch die Durchführung einer Kombination aus Waschen und Schwitzen in einem Apparat kann geeignet sein.

Die Reinheit des erhaltenen Phthalids beträgt vorzugsweise 97 bis 99,9 Gew.-%, insbesondere 98,5 bis 99,5 Gew.-%.

Die Mutterlauge und Waschlösung kann, insbesondere dann, wenn ohne Zusatz eines Hilfsstoffs kristallisiert wurde und das anodische Koppelprodukt zuvor abgetrennt wurde, ohne weitere Aufarbeitung in die Elektrolysezelle zurückgeführt werden, da sie im wesentlichen aus einem Gemisch aus Phthalid und der entsprechenden Ausgangsverbindung besteht.

Gleiches gilt auch für den Fall, daß die Kristallisation unter Zurhilfenahme von Lösungsmitteln erfolgte, die auch im Elektrolyten mitverwendet werden.

Falls das Rohphthalid aus einer Lösung kristallisiert oder mit einer Lösung gewaschen wurde, die nicht Bestandteil des Elektrolyten ist, wird das Lösungsmittel abdestilliert und der Destillationsrückstand kann anschließend in die Elektrolysezelle zurückgeführt werden.

Einer der Hauptvorteile diese Verfahrens ist darin zu sehen, daß der Anteil an unerwünschten Nebenprodukten besonders gering ist und nicht umgesetztes Ausgangsprodukt, das im wesentlichen erst als Bestandteil der Mutterlauge bei der Kristallisation des Phthalides abgetrennt werden kann, wieder dem Elektrolyten zugesetzt werden kann. Gleiches gilt für das Koppelprodukt bzw. seine Ausgangsverbindung, den anodischen Depolarisator. Das Verfahren arbeitet deshalb besonders wirtschaftlich.

### Experimenteller Teil

### Bei den prozentualen Angaben im experimentellen Teil handelt es sich um Gewichtsprozente

### Beispiel 1

In einer ungeteilten Elektrolysezelle mit einer Anodenzuführung aus Graphit und einer Kathodenzuführung aus Graphit sind Graphitringscheiben mit einer Fläche von 1,4 dm² so angeordnet, daß zehn Spalten entstehen, in denen sich eine Elektrolyse abspielen kann. Zwei weitere Graphitscheiben kontaktieren die Anoden- und die Ka-thodenzuführung. Das Prinzip der Zelle ist dem Fachmann bekannt und beispielsweise in D. Degner et al. AIChE Symposium Series No. 185, Vol. 75 S. 14 ff. beschrieben. In dieser Zelle wurden eine Lösung aus 20% Palatinol® M (o-Phthalsäuredimethylester), 13 % p-tert.-Butyltoluol und 1,2 % Methyltributylammoniummethylsul-fat, gelöst in Methanol, bei 40°C umgepumpt und kontinuierlich umgesetzt. Stromeinsatz: 6 F bezogen auf Palatinol M , Stromdichte: 2 A/dm². Phthalid hatte sich im kontinuierlichen Austrag mit einer Selektivität von 90% gebildet.

Anschließend wurde zunächst das Lösemittel Methanol im Vakuum abdestilliert. Danach wurde der Austrag im Vakuum destilliert. Nach der Fraktion des Dimethylacetals von tert.-Butylbenzaldehyd, wurde Phthalid abdestilliert. Dies erfolgte zügig, um Verglasungen infolge von Leitsalzzersetzung zu vermeiden.

Die entstandene Rohware enthielt 82 % Phthalid und 7 % Palatinol M. Das Molverhältnis (M) betrug 0,94:1. Da das Acetal des tert.-Butylbenzaldehyd nicht quantitativ von Phthalid zu trennen war, wurden auch 4% dieser Komponente gefunden.

Nach dem Abkühlen auf 25°C in einem beheizten zylindrischen Glasgefäß mit einer Glasfritte am Boden, wurden 22 bis 25 % einer flüssigen Phase mit einem Gehalt an Phthalid von 35% und einen Gehalt an Palatinol M von 24 % abgetrennt.

Die entstehende Phthalidphase hatte einen Phthalidgehalt von etwa 95 %, dieser wurde durch Abschmelzen weiterer Flüssiganteile bis 60°C auf 99% angereichert.

Ausbeute an 99%-igem Phthalid: ca. 60%

### Beispiel 2

Rohes Phthalid aus einer analogen diskontinuierlichen Elektrolyse mit einem Gehalt an Palatinol M von 13 % und einem Phthalidgehalt von 79% wurde in geschmolzener Form in die Glasapparatur gemäß Beispiel 1 eingefüllt. Nach Abkühlen auf 25°C wurde eine Flüssigphase von 28% mit einem Phthalidanteil von 41% und einem Palatinol M-Anteil von 36% erhalten (Molverhältnis (M) 0,90:1).

Nach Abschmelzen bis 60°C verblieben 53% einer 98%-igen Ware mit 0,2% Palatinol M-Restgehalt.

### Vergleichsbeispiel

In der in Beispiel 1 beschriebenen Zelle wurden eine Lösung aus 20% Palatinol M (Phthalsäuredimethylester), 12 % tert.-Butyltoluol und 1,2 % Methyltributylammoniummethylsulfat bei 50°C umgesetzt. Stromstärke 5 A.

Nach 4,4 F/Mol Palatinol M betrug der Phthalidgehalt der Lösung 11,2 Gew-%, dies entsprach einer Ausbeute von 81 %, Palatinol M hatte sich zu über 90 % umgesetzt. Das Molverhältnis (M) betrug zu diesem Zeitpunkt 0,93:1 (GC-Werte). Bei 4,8 F/Mol Palatinol M erreichte die Phthalidkonzentration mit über 12,2 Gew-% ihr Maximum, entsprechend einer Ausbeute von etwa 90%, das Molverhältnis (M) war auf 0,97:1 angestiegen. Anschließend stieg das Molverhältnis zwar weiter an, aber die Phthalidkonzentration im Elektrolyten sank: Bei 5,2 F/Mol Palatinol M lagen die Werte bei 11,1 Gew-% Gehalt Phthalid bei einem Molverhältnis (M) von 0,99:1. Erst zu diesem Zeitpunkt ist es möglich, durch Destillation eine Phthalidqualität von >98 % Reinheit zu erhalten.

Die Ausbeute an Phthalid, aufgetragen in einer Kurve als Funktion der Reaktionszeit schmiegt sich also nicht, wie es zu erwarten gewesen wäre, einem Grenzwert an sondern erreicht bei einem bestimmten Umsatz ein Maximum und fällt anschließend wieder ab. Eine hohe Wirtschaftlichkeit des Gesamtverfahrens läßt sich folglich nur dann erreichen, wenn man bei einem bestimmten Umsatz die Reaktion abbricht oder die Reaktionsmischung kontinuierlich dem Reaktionsgefäß entnimmt und von der Reaktionsmischung anschließend durch Kristallisation das Ausgangsprodukt abtrennt.

## Patentansprüche

1. Verfahren zur Herstellung von Phthaliden, indem man
I. in einem Elektrolyten gelöste Phthalsäure oder Phthalsäurederivate, bei denen die Carboxygruppen durch Einheiten ersetzt sein können, die von Carboxygruppen in einer Kondensationsreaktion ableitbar sind und eines oder mehrere der Wasserstoffatome der o-Phenylen-Einheit durch inerte Reste substituiert sein können, an einer Kathode in einer ungeteilten Elektrolysezelle reduziert,
II. wenn der Umsatz so weit fortgeschritten ist, daß das Molverhältnis (M), gebildet aus dem Anteil an Phthalid und der Summe aus dem Anteil an Phthalid und der Phthalsäure oder den Phthalsäurederivaten im Elektrolyten 0,8:1 bis 0,995:1 beträgt, den Elektrolyten aus der Elektrolysezelle austrägt und
III. die Phthalide nach destillativer Aufarbeitung des Elektrolyten aus dem Elektrolyt kristallisiert und von der Mutterlauge abtrennt.

2. Verfahren nach Anspruch 1, wobei man die destillative Aufarbeitung des Elektrolyten vornimmt, indem man das organische Lösungsmittel und anschließend eine Fraktion, die Phthalide enthält, aus dem Elektrolyten abdestilliert.

3. Verfahren nach Anspruch 1 oder 2, wobei man die destillative Aufarbeitung des Elektrolyten so vornimmt, daß man
a) in Schritt (a) im wesentlichen nur das Lösungsmittel aus den Elektrolyten abdestilliert
b) nach Schritt (a) aus dem verbleibenden Destillationsrückstand 2 weitere Fraktionen abdestilliert, von denen eine hauptsächlich die Phthalide und die andere hauptsächlich die bei der anodischen Depolarisation entstandenen Produkte enthält.

4. Verfahren zur Herstellung von Phthaliden, indem man
I. in einem Elektrolyten gelöste Phthalsäure oder Phthalsäurederivate, bei denen die Carboxygruppen durch Einheiten ersetzt sein können, die von Carboxygruppen in einer Kondensationsreaktion ableitbar sind und eines oder mehrere der Wasserstoffatome der o-Phenylen-Einheit durch inerte Reste substituiert sein können, an einer Kathode in einer ungeteilten Elektrolysezelle reduziert,
II. wenn der Umsatz so weit fortgeschritten ist, daß das Molverhältnis (M), gebildet aus dem Anteil an Phthalid und der Summe aus dem Anteil an Phthalid und der Phthalsäure oder den Phthalsäurederivaten im Elektrolyten 0,8:1 bis 0,995:1 beträgt, den Elektrolyten aus der Elektrolysezelle austrägt und
III. die Phthalide, gegebenenfalls nach destillativer Aufarbeitung des Elektrolyten, aus dem Elektrolyt kristallisiert, indem man die Kristallisation der Pthalide an einer gekühlten Fläche durchgeführt, und von der Mutterlauge abtrennt.

## Claims

1. A process for preparing phthalides by
I. reducing phthalic acid or phthalic acid derivatives, where the carboxyl groups may be replaced by units which can be derived from carboxyl groups by a condensation reaction and where one or more of the hydrogens of the o-phenylene unit may be substituted by inert radicals, at a cathode in an undivided electrolytic cell and dissolved in an electrolyte,
II. discharging the electrolyte from the electrolytic cell when the reaction has proceeded to the stage where the molar ratio (M), formed by the proportion of phthalide and the sum of the proportions of phthalide and phthalic acid or phthalic acid derivatives in the electrolyte, is from 0.8:1 to 0.995:1, and
III. crystallizing the phthalides from the electrolyte and removing them from the mother liquor, after distillative work-up of the electrolyte.

2. The process as claimed in claim 1, wherein the distillative work-up of the electrolyte is carried out by removing the organic solvent and then a fraction comprising phthalides distillatively from the electrolyte.

3. The process as claimed in claim 1 or 2, where the distillative work-up of the electrolyte is carried out by
a) removing by distillation in step (a) essentially only the solvent from the electrolytes
b) removing by distillation, after step (a), 2 additional fractions from the remaining distillation residue, one of which comprises mainly the phthalides and the other comprises mainly the products which were formed during the anodic depolarization.

4. A process for preparing phthalides by
I. reducing phthalic acid or phthalic acid derivatives, where the carboxyl groups may be replaced by units which can be derived from carboxyl groups by a condensation reaction and where one or more of the hydrogens of the o-phenylene unit may be substituted by inert radicals, at a cathode in an undivided electrolytic cell and dissolved in an electrolyte,
II. discharging the electrolyte from the electrolytic cell when the reaction has proceeded to the stage where the molar ratio (M), formed by the proportion of phthalide and the sum of the proportions of phthalide and phthalic acid or phthalic acid derivatives in the electrolyte, is from 0.8:1 to 0.995:1, and
III. crystallizing the phthalides from the electrolyte, if appropriate after distillative work-up of the electrolyte, by carrying out the crystallization of the phthalides on a cooled surface, and removing the phthalides from the mother liquor.

## Revendications

1. Procédé de préparation de phtalides, dans lequel
I. on réduit au niveau d'une cathode, dans une cellule d'électrolyse non-subdivisée, de l'acide phtalique ou des dérivés de l'acide phtalique dissous dans un électrolyte, où les groupes carboxy peuvent être remplacés par des motifs qui peuvent être obtenus à partir de groupes carboxy dans le cadre d'une réaction de condensation, et où un ou plusieurs des atomes d'hydrogène du motif o-phénylène peuvent être remplacés par des radicaux inertes,
II. quand la réaction s'est poursuivie au point que le rapport en moles (M), qui est le rapport entre la quantité de phtalide et la somme des quantités de phtalide et de l'acide phtalique ou des dérivés de l'acide phtalique dans l'électrolyte, soit de 0,8:1 à 0,995:1, on extrait l'électrolyte de la cellule d'électrolyse, et
III. après traitement de l'électrolyte par distillation, on sépare les phtalides de l'électrolyte par cristallisation, et on les sépare de la liqueur-mère.

2. Procédé selon la revendication 1, dans lequel on met en oeuvre le traitement de l'électrolyte par distillation en chassant de l'électrolyte par distillation le solvant organique puis une fraction contenant les phtalides.

3. Procédé selon la revendication 1 ou 2, dans lequel on procède au traitement de l'électrolyte par distillation de la manière suivante :
a) dans l'étape (a), ce n'est pour l'essentiel que le solvant qui est chassé de l'électrolyte par distillation,
b) après l'étape (a), on chasse du résidu de distillation restant 2 autres fractions, dont l'une contient essentiellement les phtalides, et l'autre contient essentiellement les produits qui se forment lors de la dépolarisation anodique.

4. Procédé de préparation de phtalides, dans lequel
I. on réduit au niveau d'une cathode, dans une cellule d'électrolyse non-subdivisée, de l'acide phtalique ou des dérivés de l'acide phtalique dissous dans un électrolyte, où les groupes carboxy peuvent être remplacés par des motifs qui peuvent être obtenus à partir de groupes carboxy dans le cadre d'une réaction de condensation, et où un ou plusieurs des atomes d'hydrogène du motif o-phénylène peuvent être remplacés par des radicaux inertes,
II. quand la réaction s'est poursuivie au point que le rapport en moles (M), qui est le rapport entre la quantité de phtalide et la somme des quantités de phtalide et de l'acide phtalique ou des dérivés de l'acide phtalique dans l'électrolyte, soit de 0,8:1 à 0,995:1, on extrait l'électrolyte de la cellule d'électrolyse, et
III. on sépare les phtalides de l'électrolyte par cristallisation, éventuellement après traitement de l'électrolyte par distillation, en mettant en oeuvre la cristallisation des phtalides sur une surface refroidie, et on les sépare de la liqueur-mère.
